# EUROPÄISCHE PATENTSCHRIFT

(11) **EP 1 044 664 B1**
(45) Veröffentlichungstag und Bekanntmachung des Hinweises auf die Patenterteilung: **30.06.2004**
(21) Anmeldenummer: 00107891.4
(22) Anmeldetag: 12.04.2000
(51) Int. Cl.: A61F 2/36

(54) **Profilschaft für die Verankerung einer Hüftgelenkprothese im Femur**
Profile stem for anchoring a hip joint prosthesis in the femur
Tige fémorale à profil pour l'ancrage d'une prothèse de l'articulacion de la hanche dans le fémur

(30) Priorität: 13.04.1999 DE 19916630
(43) Veröffentlichungstag der Anmeldung: 18.10.2000
(73) Patentinhaber: PLUS ENDOPROTHETIK AG, 6343 Rotkreuz (CH)
(72) Erfinder: Zweymüller, Karl, Prof. Dr. med., 1190 Wien (AT)
(74) Vertreter: Popp, Eugen, Dr.

(56) Entgegenhaltungen:
- EP-A- 0 587 955
- EP-A- 0 797 964
- FR-A- 2 768 328
- US-A- 5 456 717

## Beschreibung

Die Erfindung betrifft einen Profilschaft für die Verankerung einer Hüftgelenkprothese im Femur, mit einem Schaftabschnitt und einem am Ende des Schaftabschnitts ausgebildeten Befestigungsabschnitt, der zur Befestigung eines kugelförmigen Gelenkkopfes dient, wobei die mediale Schmalseite des Schaftabschnitts im proximalen Bereich in einen stetig gekrümmten Bogen übergeht, der im Bereich des Befestigungsabschnitts endet und wobei der Schaftabschnitt auf der lateralen Seite im proximalen Bereich in einen Trochanterflügel übergeht, an dem Kantenabschrägungsflächen (Facetten) ausgebildet sind.

Ein derartiger Profilschaft für die Verankerung einer Hüftgelenkprothese im Femur ist beispielsweise aus der US 5, 456, 717 bekannt.

Die Kantenabschrägungsflächen (Facetten) dienen dabei dazu, einen besseren Paßsitz des Profilschaftes im Trochanterflügelbereich in der Femuröffnung zu erreichen.

Es ist dabei gängige Praxis, einen Facettenwinkel von 45° bzw. 135° zu verwenden (Winkel zwischen der Ebene der lateralen Seite und der Ebene der betreffenden anliegenden Facette).

Es hat sich jedoch herausgestellt, daß eine derartige Bemessung des Facettenwinkels zu vergleichsweise großen Leerräumen an den Eckenbereichen des Trochanterflügels führt.

Die der Erfindung zugrundeliegende Aufgabe besteht darin, einen Profilschaft für die Verankerung einer Hüftgelenkprothese im Femur der eingangs genannten Art zu schaffen, bei der durch besonders einfache Maßnahmen der Leerraum im Trochanterflügelbereich wesentlich reduziert werden kann.

Diese Aufgabe wird ausgehend von dem Profilschaft der eingangs definierten Art erfindungsgemäß dadurch gelöst, daß der Winkel, der zwischen der Ebene einer Abschrägungsfläche (Facette) und der Ebene der anliegenden lateralen Seitenfläche gebildet ist, kleiner als 135° und größer als 90° ist.

Eine besonders vorteilhafte und wirksame Ausführungsform der Erfindung besteht ferner darin, daß der genannte Winkel in einem Bereich zwischen 100° und 120° liegt.

Eine insbesondere verbesserte Ausfüllung des Leerraumes im Trochanterflügelbereich kann ferner dadurch erreicht werden, daß eine Kantenabschrägungsfläche (Facette) am Übergang zwischen der lateralen Seite des Schaftes und der Anteriorseite (Breitseite) und zwischen der lateralen Seite des Schaftes und der Posteriorseite (Breitseite) des Schaftes ausgebildet ist.

Die Erfindung ist ferner dadurch gekennzeichnet, daß die Kantenabschrägungsflächen als ungleichschenklige Facetten ausgebildet sind.

Eine besonders vorteilhafte Ausführungsform der Erfindung besteht bei einem Protilschaft, bei dem der Schaftabschnitt auf der lateralen Seite in Form einer stetig gekrümmten Kurve in den Trochanterflügel übergeht, darin, daß innerhalb des Bereiches der stetig gekrümmten Kurve die Kantenabschrägungsflächen jeweils in Richtung vom Schaftbereich zum Trochanterbereich von einer gleichschenkligen Facette zu einer ungleichschenkligen Facette wechseln.

Dabei kann der Wechsel von gleichschenkliger Facette zu ungleichschenkliger Facette in Form eines stufenlosen Übergangs erfolgen oder gemäß einer alternativen Ausführungsform kann der Wechsel von gleichschenkliger Facette zu ungleichschenkliger Facette in Form einer Stufe erfolgen.

Im folgenden wird die Erfindung anhand von Ausführungsbeispielen unter Hinweis auf die Zeichnung näher erläutert. Es zeigen:
- Fig. 1: eine perspektivische Ansicht eines Profilschaftes für die Verankerung einer Hüftgelenkprothese im Femur mit Merkmalen nach der Erfindung;
- Fig. 2: eine Schnittansicht in teilweise weggebrochener Darstellung eines Profilschaftes in eingesetztem Zustand mit Merkmalen nach der Erfindung;
- Fig. 3: eine vergrößerte Darstellung eines Eckbereiches des Trochanterflügels in eingesetztem Zustand; und
- Fig. 4: eine Teilansicht des oberen Abschnitts des Profilschaftes mit Merkmalen nach der Erfindung.

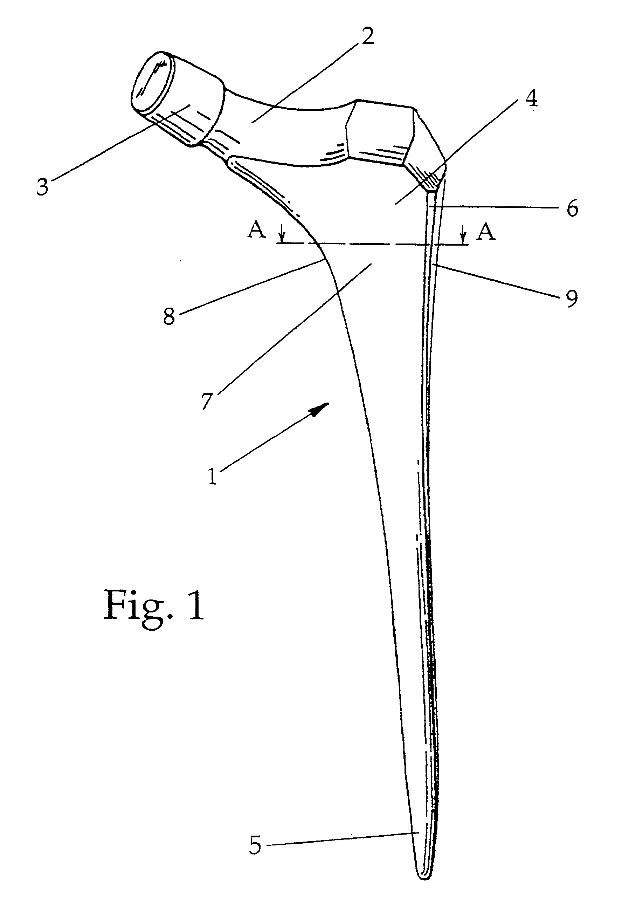

Fig. 1 zeigt eine perspektivische Ansicht eines Ausführungsbeispiels eines Profilschaftes, bei dem die Merkmale nach der vorliegenden Erfindung realisiert sind. Der gezeigte Profilschaft ist allgemein mit 1 bezeichnet und umfaßt ein unteres distales schmales Ende 5 und einen sich nach oben hin erweiternden Abschnitt 7, der im oberen Endbereich in einen Befestigungsabschnitt 2 übergeht, wobei der Befestigungsbereich 2 in einen kegelstumpfförmigen Abschnitt 3 übergeht, der zur Befestigung eines kugelförmigen Gelenkkopfes dient. Auf der medialen schmalen Seite verläuft der Schaft im proximalen Bereich in Form einer stetig gekrümmten Kurve 8, die schließlich im Bereich des Befestigungsabschnitts 2 endet.

Auf der lateralen Seite geht der Schaftabschnitt im proximalen Bereich in einen Trochanterflügel 4 über, an dem Kanter abschrägungsflächen 6 (Facetten) ausgebildet sind.

Bei der herkömmlichen Konstruktion liegt der Winkel zwischen der Ebene der Kantenabschrägungsfläche 6 und der Ebene der anliegenden lateralen Seitenfläche 9 bei 135° (bzw. 45°).

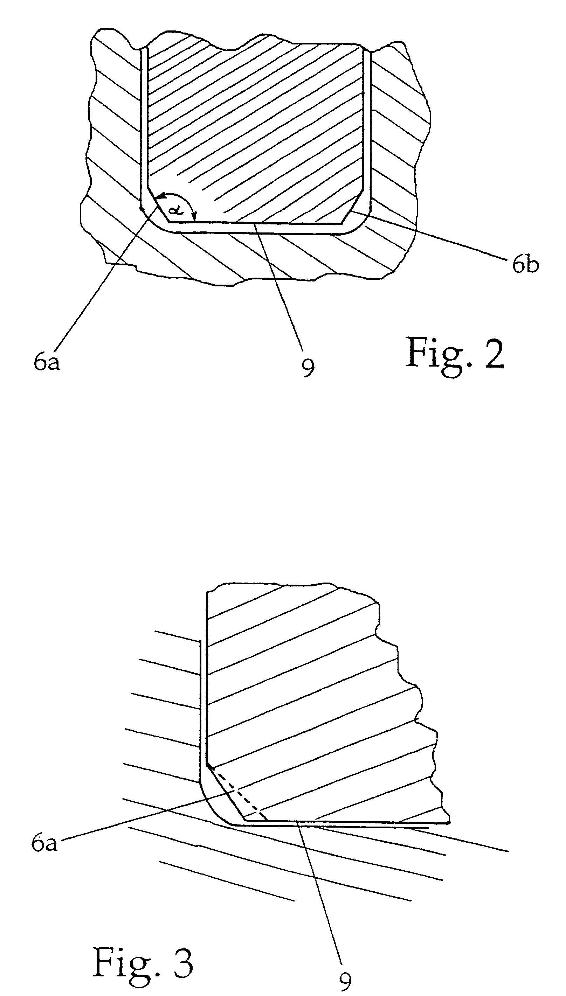

Wie sich aus Fig. 2 entnehmen läßt, die eine Teilansicht einer Schnittdarstellung gemäß einer Linie A-A in Fig. 1 veranschaulicht (in eingesetztem Zustand), verlaufen die Kantenabschrägungsflächen 6a und 6b in bezug auf die Ebene der lateralen Seitenfläche 9 im Vergleich zum Stand der Technik steiler, das heißt der Winkel α, der zwischen der Ebene einer Abschrägungsfläche 6a oder 6b und der Ebene der jeweils anliegenden lateralen Seitenfläche 9 gebildet ist, ist kleiner als 135° und größer als 90°.

Eine besonders bevorzugte Ausführungsform der Erfindung besteht darin, daß der Winkel α in einem Bereich zwischen 100° und 120° liegt.

In Fig. 3 ist der linke Kantenbereich als vergrößerte Teildarstellung ebenfalls im Schnitt gezeigt, wobei die Ausführungsform gemäß dem Stand der Technik in Form einer strichlierten Linie eingezeichnet ist. Es läßt sich aus Fig. 3 erkennen, daß bei der erfindungsgemäßen Konstruktion der zwischen dem Trochanterflügelbereich und dem umgebende Knochen befindliche Leerraum sehr viel kleiner ist als vergleichsweise bei der herkömmlichen Konstruktion, das heißt es wird bei der erfindungsgemäßen Konstruktion eine bessere Ausfüllung des Leerraumes im Trochanterflügelbereich erreicht.

Es sei darauf hingewiesen, daß bei der erfindungsgemäßen Konstruktion jeweils eine Kantenabschrägungsfläche (Facette) am Übergang zwischen der lateralen Seite des Schaftes und des Trochanterflügels und der Anteriorseite und zwischen der lateralen Seite des Schaftes und des Trochanterflügels und der Posteriorseite des Schaftes ausgebildet ist.

Die Kantenabschrägungsflächen sind bei der erfindungsgemäßen Konstruktion insbesondere als ungleichschenklige Facetten ausgebildet.

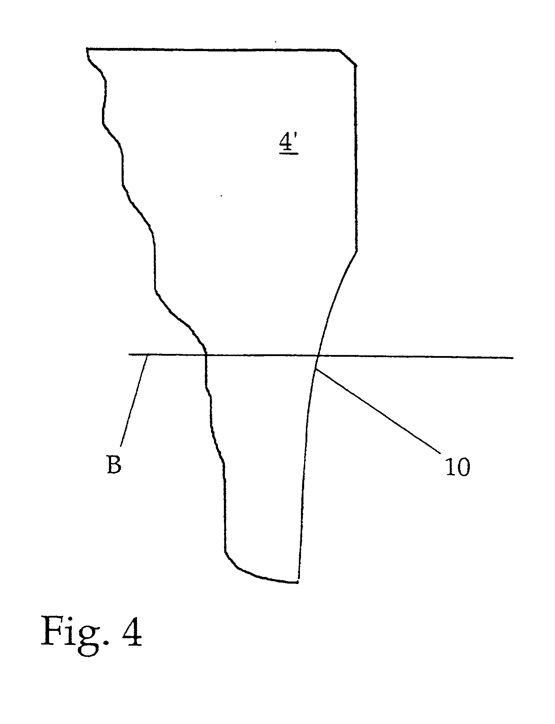

Eine weitere Ausführungsform der vorliegenden Erfindung ist in Fig. 4 schematisch dargestellt. Fig. 4 zeigt den oberen Abschnitt des Profilschaftes mit dem Trochanterflügel 4' in Form eines vergrößerten Ausschnitts. Bei der in Fig. 4 gezeigten Ausführungsform geht der Schaftabschnitt im proximalen Bereich auf der lateralen Seite in Form einer stetig gekrümmten Kurve 10 in den Trochanterflügel 4' über.

Das wesentliche dieser Ausführungsform besteht darin, daß innerhalb des Bereiches der stetig gekrümmten Kurve 10 etwa in Höhe der Linie B die Kantenabschrägungsflächen jeweils in Richtung vom Schaftbereich zum Trochanterbereich von einer gleichschenkligen Facette zu einer ungleichschenkligen Facette wechseln.

Diese Ausführungsform bietet den besonderen Vorteil, daß die ungleichschenklige Facette maschinell einfacher, beispielsweise durch Drehung des Schaftes bei der Herstellung um die Schaftlängsachse oder eine entsprechende Drehung des bearbeitenden Werkzeuges ausgebildet werden kann.

Eine vorteilhafte Ausgestaltung der in Fig. 4 gezeigten Ausführungsform besteht darin, daß der Wechsel von gleichschenkliger Facette zu ungleichschenkliger Facette in Form eines stufenlosen Übergangs erfolgt.

Eine weitere Ausführungsform besteht darin, daß der Wechsel von gleichschenkliger Facette zu ungleichschenkliger Facette in Form einer Stufe erfolgt.

Es sei darauf hingewiesen, daß die vorliegende Erfindung nicht auf die gezeigten Ausführungsbeispiele beschränkt ist und daß eine Reihe von Abwandlungen und Änderungen vorgenommen werden können, ohne dadurch den Rahmen der vorliegenden Erfindung zu verlassen.

So besteht beispielsweise die Möglichkeit, nicht nur einen Übergangsbereich von gleichschenkliger Facette zu ungleichschenkliger Facette in der oben erläuterten Weise auszubilden, sondern mehrere derartige Übergänge innerhalb des Bereiches der gekrümmten Kurve auszubilden.

### Bezugszeichenliste

- 1 =: Profilschaft
- 2 =: Befestigungsabschnitt
- 3 =: kegelstumpfförmiger Abschnitt
- 4 =: Trochanterflügelbereich
- 5 =: distales Ende
- 6 =: Kantenabschrägungsfläche
- 6a, 6b =: Kantenabschrägungsfläche
- 7 =: sich erweiternder Abschnitt
- 8 =: stetig gekrümmter Bogen
- 9 =: laterale Seitenfläche
- 10 =: stetig gekrümmter Bogen
- A-A =: Linie
- 4' =: Trochanterflügel
- α =: Winkel

## Patentansprüche

1. Profilschaft (1) für die Verankerung einer Hüftgelenkprothese im Femur, mit einem Schaftabschnitt (7) und einem am Ende des Schaftabschnitts (7) ausgebildeten Befestigungsabschnitt (2), der zum Befestigen eines kugelförmigen Gelenkkopfes dient, wobei die mediale Schmalseite des Schaftabschnittes im proximalen Bereich in einen stetig gekrümmten Bogen (8) übergeht, der im Bereich des Befestigungsabschnitts (2) endet und wobei der Schaftabschnitt (7) auf der lateralen Seite im proximalen Bereich in einen Trochanterflügel (4) übergeht, an dem Kantenabschrägungsflächen (6a, 6b) bzw. Facetten ausgebildet sind,
**dadurch gekennzeichnet, daß**
der Winkel (α), der zwischen der Ebene einer Abschrägungsfläche bzw. Facette (6a, 6b) und der Ebene der anliegenden lateralen Seitenfläche (9) gebildet ist, kleiner ist als 135° und größer ist als 90°.

2. Profilschaft nach Anspruch 1,
**dadurch gekennzeichnet, daß** der Winkel (α) in einem Bereich zwischen 100° und 120° liegt.

3. Profilschaft nach Anspruch 1 oder 2,
**dadurch gekennzeichnet, daß**
eine Kantanabschrägungsfläche (6a, 6b) im Bereich des Trochanterflügels auf der lateralen Seite (9) des Schaftes am Übergang zwischen der einen Breitseite, nämlich der Anteriorseite des Trochanterflügels und der lateralen Seite und zwischen der lateralen Seite (9) des Schaftes und der anderen Breitseite, nämlich der Posteriorseite des Trochanterflügels (4; 4') ausgebildet ist.

4. Profilschaft nach einem der Ansprüche 1 bis 3,
**dadurch gekennzeichnet, daß** die Kantenabschrägungsflächen (6a, 6b) als ungleichschenklige Facetten ausgebildet sind.

5. Profilschaft nach einem der Ansprüche 1 bis 3, bei dem der Schaftabschnitt auf der lateralen Seite in Form einer stetig gekrümmten Kurve in den Trochanterflügel (4') übergeht,
**dadurch gekennzeichnet, daß** innerhalb des Bereiches der stetig gekrümmten Kurve (10) die Kantenabschrägungsflächen jeweils in Richtung vom Schaftbereich zum Trochanterbereich von einer gleichschenkligen Facette zu einer ungleichschenkligen Facette wechseln.

6. Profilschaft nach Anspruch 5,
**dadurch gekennzeichnet, daß** der Wechsel von gleichschenkliger Facette zu ungleichschenkliger Facette in Form eines stufenlosen Übergangs erfolgt.

7. Profilschaft nach Anspruch 5,
**dadurch gekennzeichnet, daß** der Wechsel von gleichschenkliger Facette zu ungleichschenkliger Facette in Form einer Stufe erfolgt.

## Claims

1. A profiled stem (1) for anchoring a hip joint prosthesis in the femur, comprising a stem portion (7) and a fixing portion (2) which is formed at the end of the stem portion (7) and serves to fix a spherical joint head, wherein the medial narrow side of the stem portion merges in the proximal region into a continuously bent curve (8) ending in the region of the fixing portion (2), and wherein the stem portion (7) merges on the lateral side in the proximal region into a trochanter wing (4) on which are formed edge chamfers (6a, 6b) or facets, **characterised in that** the angle (α), which is formed between the plane of a chamfer or facet (6a, 6b) and the plane of the adjacent lateral side surface (9), is smaller than 135° and greater than 90°.

2. A profiled stem according to claim 1, **characterised in that** the angle (α) lies in a range between 100° and 120°.

3. A profiled stem according to claim 1 or 2, **characterised in that** an edge chamfer (6a, 6b) is formed in the region of the trochanter wing on the lateral side (9) of the stem at the transition between the one broad side, namely the anterior side of the trochanter wing, and the lateral side and between the lateral side (9) of the stem and the other broad side, namely the posterior side of the trochanter wing (4; 4').

4. A profiled stem according to any one of claims 1 to 3, **characterised in that** the edge chamfers (6a, 6b) are formed as unequal-sided facets.

5. A profiled stem according to any one of claims 1 to 3, wherein the stem portion merges on the lateral side into the trochanter wing (4') by way of a continuously bent curve, **characterised in that**, within the region of the continuously bent curve (10), the edge chamfers each change from an equal-sided facet to an unequal-sided facet in the direction extending from the stem region towards the trochanter region.

6. A profiled stem according to claim 5, **characterised in that** the change from equal-sided facet to unequal-sided facet takes place by way of a stepless transition.

7. A profiled stem according to claim 5, **characterised in that** the change from equal-sided facet to unequal-sided facet takes place by way of a step.

## Revendications

1. Tige profilée (1) pour l'ancrage d'une prothèse de l'articulation de la hanche dans le fémur, avec une portion de tige (7) et une portion d'ancrage ménagée à l'extrémité de la portion de tige (2), qui sert à la fixation d'une tête d'articulation de forme sphérique, la face étroite médiane de la portion de tige dans le domaine proximal se prolongeant en un arc (8) arrondi en continu qui se termine dans la zone de la portion d'ancrage et la portion de tige (7) se prolongeant sur la face latérale dans la région proximale dans une aile du trochanter (4) sur laquelle sont formées des surfaces en biseaux (facettes) (6a, 6b), **caractérisée en ce que** l'angle (α) qui est formé entre le plan d'une surface en biseau ou facette (6a, 6b) et le plan des faces latérales adjacentes (9) est inférieur à 135° et supérieur à 90°.

2. Tige profilée selon la revendication 1, **caractérisée en ce que** l'angle (α) est compris dans un intervalle de 100° à 120°.

3. Tige profilée selon la revendication 1 ou 2, **caractérisée en ce qu'**une surface en biseau (6a, 6b) est formée dans la zone de l'aile du trochanter sur la face latérale (9) de la tige au niveau de la transition entre une face large, à savoir la face antérieure de l'aile du trochanter et la face latérale et entre la face latérale (9) de la tige et l'autre face large, à savoir la face postérieure de l'aile du trochanter (4, 4').

4. Tige profilée selon l'une quelconque des revendications 1 à 3, **caractérisée en ce que** les surfaces en biseaux (6a, 6b) sont constituées en facettes à côtés non identiques.

5. Tige profilée selon l'une quelconque des revendications 1 à 3, dans laquelle la portion de tige sur la face latérale se prolonge sous la forme d'un arc recourbé de manière continue dans l'aile du trochanter (4'), **caractérisée en ce que**, dans la région de l'arc recourbé de manière continue (10), les surfaces en biseaux alternent respectivement en direction de la zone de tige vers la zone du trochanter, d'une facette à côtés identiques à une facette à côtés non identiques.

6. Tige profilée selon la revendication 5, **caractérisée en ce que** l'alternance de la facette à côtés identiques à la facette à côtés non identiques s'effectue en une transition sans palier.

7. Tige profilée selon la revendication 5, **caractérisée en ce que** l'alternance de la facette à côtés identiques à la facette à côtés non identiques s'effectue sous la forme d'un palier.
